(19) Europäisches Patentamt
European Patent Office
Office européen des brevets

(11) Veröffentlichungsnummer : **0 019 726**
**B1**

# (12) EUROPÄISCHE PATENTSCHRIFT

(45) Veröffentlichungstag der Patentschrift : **26.09.84**

(51) Int. Cl.³ : **C 07 D207/452**, C 08 K 5/43// G03F1/02

(21) Anmeldenummer : **80102258.3**

(22) Anmeldetag : **25.04.80**

(54) **N-Azidosulfonylaryl-maleinimide sowie deren Verwendung.**

(30) Priorität : **16.05.79 DE 2919823**

(43) Veröffentlichungstag der Anmeldung : **10.12.80 Patentblatt 80/25**

(45) Bekanntmachung des Hinweises auf die Patenterteilung : **26.09.84 Patentblatt 84/39**

(84) Benannte Vertragsstaaten : **BE CH DE FR GB IT LI NL SE**

(56) Entgegenhaltungen :
**GB-A- 1 030 886**
**US-A- 3 562 269**
**Chemical Abstracts, Band 84, Nr. 19, 10. Mai 1976, Columbus, Ohio, USA A.V. EL'TSOV et al. "On the photolytic crosslinking of polymers using disulfonylazides", Seite 33, Abstract Nr. 136481z**
**Die Akte enthält technische Angaben, die nach dem Eingang der Anmeldung eingereicht wurden und die nicht in dieser Patentschrift enthalten sind.**

(73) Patentinhaber : **Siemens Aktiengesellschaft Berlin und München Wittelsbacherplatz 2 D-8000 München 2 (DE)**

(72) Erfinder : **Rubner, Roland, Dr.**
**Buchenring 15**
**D-8551 Röttenbach (DE)**
Erfinder : **Kühn, Eberhard**
**Bergstrasse 32**
**D-8551 Hemhofen (DE)**
Erfinder : **Ahne, Hellmut, Dr.**
**Heidestrasse 6**
**D-8551 Röttenbach (DE)**

**EP 0 019 726 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Cyclische N-Azido sulfonylarylimide sind bereits aus C.A. *84* (1976), 136481Z sowie US-A-3562269 bekannt ; es ist auch bekannt, daß solche Verbindungen vernetzende Eigenschaften aufweisen und sich daher für die phototechnische Herstellung von Reliefstrukturen als Photoinitiatoren eignen.

Die Erfindung betrifft neue Arylsulfonylazide sowie die Verwendung dieser Verbindungen.

Insbesondere betrifft die Erfindung N-Azidosulfonylarylmaleinimide der allgemeinen Formel

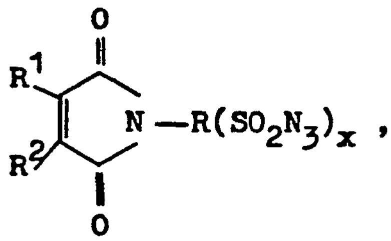

worin R = Aryl und x = 1 oder 2 ist und $R^1$ und $R^2$ die Bedeutung H, $CH_3$ und Cl haben und gleich oder verschieden sein können, mit der Maßgabe, daß $CH_3$ und Cl nicht nebeneinander vorliegen. Unter « Aryl » werden dabei aromatische Kohlenwasserstoffreste, wie Phenyl und Naphthyl, verstanden. Eine bevorzugte Verbindung ist N-(4-Azidosulfonylphenyl)-maleinimid. Weitere Verbindungen sind beispielsweise 2-(N-Maleinimido)-naphthyl-5-sulfonylazid und 2-(N-Maleinimido)-naphthyl-6.8-bissulfonylazid.

Die neuen Verbindungen werden beispielsweise aus Acetylaminobenzolsulfonsäurechloriden über die entsprechenden Aminobenzolsulfonylazide (siehe : « Die Angewandte Makromolekulare Chemie », Bd. 9, 1969, S. 1 ff) auf folgendem Weg hergestellt :

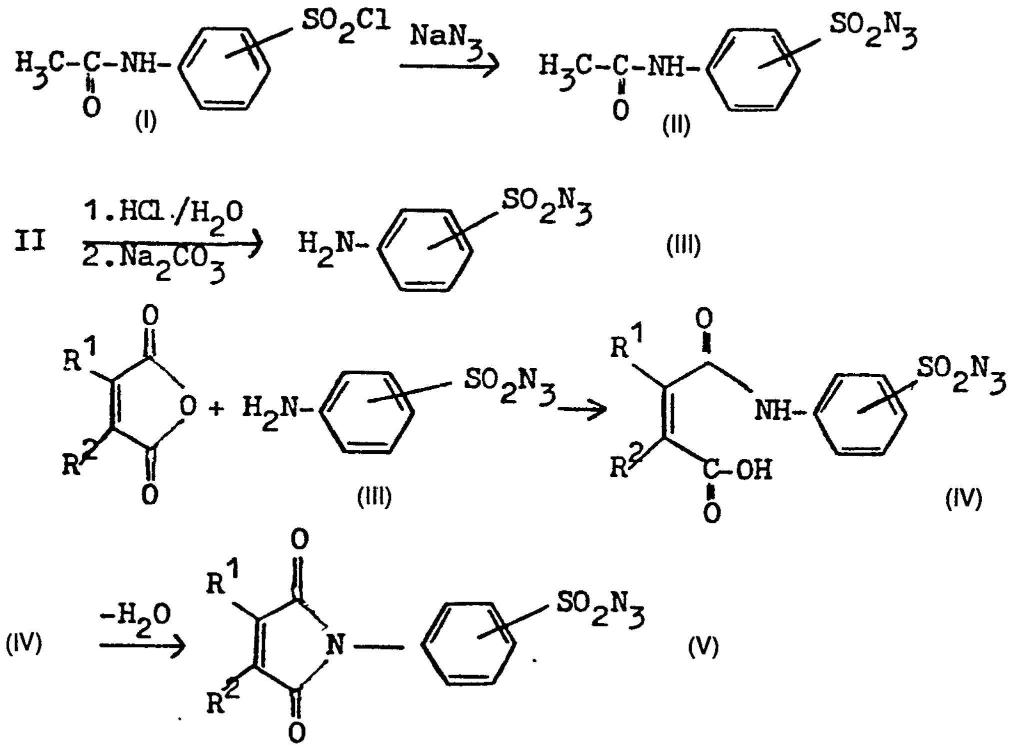


Die erfindungsgemäßen Verbindungen eignen sich vorteilhaft als Photoinitiatoren bei der phototechnischen Herstellung von Reliefstrukturen aus Gemischen, die olefinisch ungesättigte Polymere und — als Photoinitiatoren — Azide enthalten. Ein derartiges Verfahren ist in der gleichzeitig eingereichten europäischen Patentanmeldung Nr 80102259.1, « Verfahren zur phototechnischen Herstellung von Reliefstrukturen » beschrieben. Darüber hinaus können die erfindungsgemäßen Verbindungen vorteilhaft auch als vernetzungsverstärkende Agentien bei der radikalischen Vernetzung von thermoplastischen Polymeren eingesetzt werden. Beispielhaft sei hier die peroxidische Vernetzung von Polyolefinen, insbesondere Äthylen-Vinylacetat-Copolymeren (EVA), genannt.

Im folgenden soll die Erfindung — anhand der Herstellung von Zwischen- und Endprodukten — noch näher erläutert werden.

1. 4-Acetylaminobenzolsulfonylazid

233,5 g 4-Acetylaminobenzolsulfonsäurechlorid (ca. 1 Mol) werden in der Kälte (bei 2 bis 3 °C) und unter Rühren in eine Lösung von 70 g Natriumazid (ca. 1 Mol) in 800 g Wasser eingetragen. Anschließend wird noch 18 Stunden bei Raumtemperatur gerührt und dann wird die Temperatur für eine Stunde auf 40 °C gehalten ; schließlich wird abgesaugt und mit reichlich Wasser gewaschen, wobei eine braune Substanz erhalten wird.

2. 4-Aminobenzolsulfonylazid-Hydrochlorid

Das nach (1) erhaltene 4-Acetylaminobenzolsulfonylazid wird mit 400 g 32 %iger wäßriger Salzsäure verrührt und die Mischung innerhalb von 20 Minuten auf eine Temperatur von 95 °C gebracht ; dabei geht die Substanz vollständig in Lösung. Aus der heißen salzsauren Lösung kristallisiert das Hydrochlorid des 4-Aminobenzolsulfonylazids beim Abkühlen aus und wird abfiltriert.

3. 4-Aminobenzolsulfonylazid

Aus dem nach (2) erhaltenen Hydrochlorid wird die freie Base in der üblichen Weise mit wäßriger Sodalösung (ca. 100 g $Na_2CO_3$ in 500 ml Wasser) als schnell erstarrendes Öl erhalten. Die Ausbeute beträgt 130 bis 150 g (60 bis 80 % der Theorie). Das Produkt wird mit Wasser gewaschen und im Trockenschrank über $P_2O_5$ getrocknet ; Schmelzpunkt : 35 bis 36 °C.

4. N-(4-Azidosulfonylphenyl)-maleinsäuremonoamid

100 g Maleinsäureanhydrid werden in 1 l Dichlormethan gelöst und zu dieser Lösung werden 198 g des nach (3) hergestellten 4-Aminobenzolsulfonylazids (ca. 1 Mol), gelöst in 400 ml Dichlormethan, zugetropft. Das Reaktionsgemisch wird zwei Stunden bei Raumtemperatur stehengelassen, dann wird die Lösung am Rotationsverdampfer bis auf ca. 200 ml eingedampft. Das dabei erhaltene hellbraune Produkt wird abgekühlt, dann wird filtriert, mit wenig Dichlormethan gewaschen und schließlich getrocknet.

5. N-(4-Azidosulfonylphenyl)-maleinimid

280 g des nach (4) hergestellten Maleinsäuremonoamids werden in 1 l Dichlormethan suspendiert und zu dieser Suspension wird bei 0 °C eine Mischung aus 153 g 1-Hydroxybenzotriazol $H_2O$ (1 Mol) und 230 g N,N'-Dicyclohexylcarbodiimid (ca. 1,1 Mol) gegeben. Nach 12 bis 15 Stunden wird der ausgefallene Harnstoff abfiltriert und das Dichlormethan abgedampft. Durch Zerreiben mit Petroläther wird das überschüssige N,N'-Dicyclohexylcarbodiimid entfernt. Das dabei erhaltene Produkt wird säulenchromatographisch an Kieselgel mit Essigester oder einer Mischung aus Essigester und Dichlormethan als Laufmittel gereinigt.

Das in der vorstehend beschriebenen Weise hergestellte N-(4-Azidosulfonylphenyl)-maleinimid fällt in Form schwach gelblicher Nadeln an, die in Aceton, Chloroform, Dimethylacetamid und anderen gängigen Lösungsmitteln löslich sind ; Schmelzpunkt : 120 °C (unter Zersetzung).

Elementaranalyse :

ber. C 43,16 % H 2,17 % N 20,14 %

gef. C 42,82 % H 2,16 % N 19,80 %

Das IR-Spektrum zeigt folgende charakteristische Banden :

$—N_3$ :2 130 cm$^{-1}$ bzw. 4,69 μm ;

—CO—N :1 775 cm$^{-1}$ bzw. 5,63 μm und 1 730 cm$^{-1}$ bzw. 5,82 μm ;

$>C=C<$(aromatisch) :1 595 cm$^{-1}$ bzw. 6,28 μm und 1 493 cm$^{-1}$ bzw. 6,68 μm.

**Ansprüche**

1. N-Azidosulfonylaryl-maleinimide der allgemeinen Formel

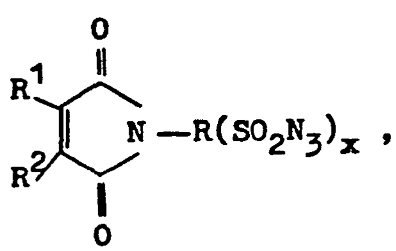

worin R = Aryl und x = 1 oder 2 ist und $R^1$ und $R^2$ die Bedeutung H, $CH_3$ und Cl haben und gleich oder verschieden sein können, mit der Maßgabe, daß $CH_3$ und Cl nicht nebeneinander vorliegen.

2. N-(4-Azidosulfonylphenyl)-maleinimid.

3. Verwendung von Verbindungen gemäß Anspruch 1 oder 2 als vernetzungsverstärkende Agentien bei der radikalischen Vernetzung von thermoplastischen Polymeren.

**Claims**

1. N-azidosulphonylaryl-maleimides of the general formula

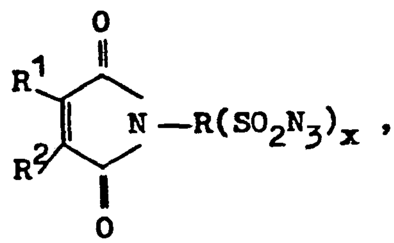

where R = Aryl and X = 1 or 2 and $R^1$ and $R^2$ signify H, $CH_3$ and Cl and can be the same or different, provided that $CH_3$ and Cl are not arranged next to one another.

2. N-(4-azidosulphonylphenyl)-maleimide.

3. The use of compounds according to Claim 1 or Claim 2 as cross-linkage reinforcing agents in the free radical cross-linking of thermoplastic polymers.

**Revendications**

1. N-azidosulfonylaryl-maléimides de formule développée.

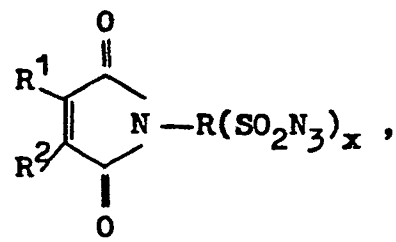

dans laquelle R = aryle et x = 1 ou 2 et $R^1$ et $R^2$ ont la signification H, $CH_3$ et Cl et peuvent être identiques ou différents, $CH_3$ et Cl ne se trouvent pas l'un à côté de l'autre.

2. Le N-(4-azidosulfonylphényl)-maléimide.

3. Utilisation de composés suivant la revendication 1 ou 2, comme agents intensifiant la réticulation, lors de la réticulation radicalaire de polymères thermoplastiques.